# EUROPEAN PATENT APPLICATION

(11) **EP 3 006 033 A1**
(43) Date of publication of application: **13.04.2016**
(21) Application number: 14807629.2
(22) Date of filing: 06.06.2014
(51) Int. Cl.: A61K 31/683, A61P 35/00, A61P 43/00

(54) **ANTICANCER DRUG**

(30) Priority: 07.06.2013 JP 2013121142
(71) Applicant: KTN Biotec, Inc., Hirakata-shi, Osaka 573-1171 (JP)
(72) Inventor: NISHIZAKI, Tomoyuki, Kobe-shi Hyogo 651-1223 (JP); NAKANO, Takashi, Kobe-shi Hyogo 658-0001 (JP); TANAKA, Akito, Toyonaka-shi Osaka 560-0012 (JP); SHIMIZU, Tadashi, Kobe-shi Hyogo 651-0093 (JP); KANNO, Takeshi, Nishinomiya-shi Hyogo 662-0841 (JP)
(74) Representative: von Kreisler Selting Werner - Partnerschaft von Patentanwälten und Rechtsanwälten mbB
(86) International application number: PCT/JP2014/065042
(87) International publication number: WO 2014/196621

(57) **Abstract**

An anti-cancer agent and a cancer cell death inducing agent, containing an inositol phospholipid compound, particularly 1,2-O-bis-[8-{2-(2-pentyl-cyclopropylmethyl)-cyclopropyl}-octanoyl]-sn-glycerol-3-phosphatidyl-inositol, as an active ingredient.

## Description

### Technical Field

The present invention relates to an anti-cancer agent containing an inositol phospholipid compound having a cancer cell proliferation-suppressive action, particularly an inositol phospholipid compound containing an unsaturated fatty acid derivative, as an active ingredient.

### Background Art

At present, cancer is the leading causal disease of death and a basic treatment method thereof has been demanded. For this end, many anti-cancer agents such as bleomycin, cisplatin, neocarzinostatin and the like have been developed. However, these anti-cancer agents could not provide a sufficient anti-cancer effect, since they have high toxicity and are less oriented to cancer. Accordingly, various attempts have been made to make these anti-cancer agents more oriented to cancer, and one of such attempts is the development of a liposome preparation for administration of an anti-cancer agent enclosed inside the liposome. Liposome preparation is a preparation including a drug inside a particle having a bilayer membrane structure formed of phospholipid, and detailed analyses as regards cancer cell targeting have been performed.

Akt is one of the target molecules of anti-cancer agents and the metabolism of inositol phospholipid is involved in the activation thereof. That is, PI3 kinase phosphorylates phosphatidylinositol 4,5-diphosphate to produce phosphatidylinositol 3,4,5-triphosphate (PIP3), PIP3 binds to 3-phosphoinositide-dependent protein kinase (PDK) to activate PDK, and further, PDK phosphorylates Akt to activate Akt. While there has been made an attempt to indirectly control the function of Akt by suppressing synthesis of inositol phospholipids such as PIP3 and the like, the anti-cancer action of inositol phospholipid itself is hardly known.

Phospholipid is a major lipid constituting the biological membrane system, and is divided into glycerolphospholipid having a glycerol skeleton, and sphingophospholipid having a sphingosine skeleton. Furthermore, depending on the kind of the hydrophilic moiety, glycerolphospholipids are divided into phospholipid classes of phosphatidylcholine, phosphatidylethanolamine, phosphatidylserine, phosphatidylinositol, phosphatidylglycerol, cardiolipin, phosphatidic acid and the like.

As the functions of phospholipids, there have been reported, besides the role of a membrane-constituting component that divides cells and cell organelles, that inositol phospholipid has a known role as an intercellular signaling pathway by phospholipase C and an anchor of protein-membrane; phosphatidylserine regulates the activities of blood coagulation protein, protein kinase C and the like; sphingomyelin pathway is involved in the regulation of activity of protein kinase C and cell apoptosis; phosphatidylcholine pathway relates to the maintenance of arachidonic acid which is an inflammatory mediator and signal transduction pathway by phospholipase D; platelet-activating factor which is alkyl ether phospholipid shows platelet activation, blood vessel permeability, leukocyte migration activity; and the like, and the functions provided by the classes thereof vary widely.

For example, 1,2-dilinoleoyl-sn-glycero-3-phosphocholine and 1-palmitoyl-2-oleoyl-sn-glycero-3-phosphocholine have been reported to improve spatial learning disorder and memory disorder induced by scopolamine, or mild cognitive impairment and dementia (non-patent documents 1, 2).

Also, phosphatidylethanolamine is a phospholipid, which is one of the main components of biological membrane, and is being marketed along with phosphatidylserine and the like as health foods. Of phosphatidylethanolamines, particularly, dilinoleoyl phosphatidylethanolamine (containing two linoleic acids as fatty acids) has been reported to have cell death induction suppressive activity, particularly, endoplasmic reticulum stress suppressive activity and, due to such activity, dilinoleoyl phosphatidylethanolamine can be used for pharmaceutical application, particularly for the prophylaxis and/or treatment of neurodegenerative disease (patent document 1).

### [Document List]

### Patent Documents

patent document 1: JP-A-2005-247728

### [non-patent document]

non-patent document 1: Yaguchi T, Nagata T, Nishizaki T. Dilinoleoylphosphatidylcholine ameliorates scopolamine-induced impairment of spatial learning and memory by targeting alpha-7 nicotinic ACh receptors. Life Sci 2009; 84:263-6
non-patent document 2: Yaguchi T, Nagata T, Nishizaki T. 1-Palmitoyl-2-oleoyl-sn-glycero-3-phosphocholine improves cognitive decline by enhancing long-term depression. Behav Brain Res 2009; 204:129-32

### SUMMARY OF THE INVENTION

### Problems to be Solved by the Invention

The present invention aims to provide an anti-cancer agent having a novel action mechanism.

### Means of Solving the Problems

The present inventors have conducted intensive studies in an attempt to achieve the above-mentioned object and found that inositol phospholipid (phosphatidylinositol) has a superior cancer cell proliferation suppressive action. Furthermore, they have confirmed a superior anti-cancer action of inositol phospholipid having, as fatty acid, unsaturated fatty acid, particularly unsaturated fatty acid having a cyclopropane ring, which resulted in the completion of the present invention. Accordingly, the present invention is as described below.
[1] An anti-cancer agent comprising an inositol phospholipid compound as an active ingredient.
[2] The agent of the above-mentioned [1], wherein the fatty acid constituting the inositol phospholipid compound is an unsaturated fatty acid.
[3] The agent of the above-mentioned [2], wherein the unsaturated fatty acid is oleic acid.
[4] The agent of the above-mentioned [1], wherein the fatty acid constituting the inositol phospholipid compound is an unsaturated fatty acid having a double bond converted to a cyclopropane ring.
[5] The agent of the above-mentioned [4], wherein the unsaturated fatty acid is 8-[2-(2-pentyl-cyclopropylmethyl)-cyclopropyl]-octanoic acid (DCP-LA).
[6] The agent of the above-mentioned [1], wherein the fatty acid constituting the inositol phospholipid compound is a saturated fatty acid.
[7] The agent of the above-mentioned [6], wherein the saturated fatty acid is palmitic acid.
[8] The agent of the above-mentioned [1], wherein the inositol phospholipid compound is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(1'-myo-inositol) (DO-PI), or 1,2-dipalmitoyl-sn-glycerol-3-phospho-(1'-myo-inositol) (DP-PI).
[9] The agent of any of the above-mentioned [1] - [8], wherein the treatment target cancer is at least one kind selected from the group consisting of malignant pleural mesothelioma, lung cancer, gastric cancer and breast cancer.
[10] A cancer cell death-inducing agent comprising an inositol phospholipid compound as an active ingredient.
[11] The agent of the above-mentioned [10], wherein the fatty acid constituting the inositol phospholipid compound is an unsaturated fatty acid.
[12] The agent of the above-mentioned [11], wherein the unsaturated fatty acid is oleic acid.
[13] The agent of the above-mentioned [10], wherein the fatty acid constituting the inositol phospholipid compound is an unsaturated fatty acid having a double bond converted to a cyclopropane ring.
[14] The agent of the above-mentioned [13], wherein the unsaturated fatty acid is 8-[2-(2-pentyl-cyclopropylmethyl)-cyclopropyl]-octanoic acid (DCP-LA).
[15] The agent of the above-mentioned [10], wherein the fatty acid constituting the inositol phospholipid compound is saturated fatty acid.
[16] The agent of the above-mentioned [15], wherein the saturated fatty acid is palmitic acid.
[17] The agent of the above-mentioned [10], wherein the inositol phospholipid compound is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(1'-myo-inositol) (DO-PI), or 1,2-dipalmitoyl-sn-glycerol-3-phospho-(1'-myo-inositol) (DP-PI).
[18] The agent of any of the above-mentioned [10] - [17], wherein the cancer cell is a cell of at least one kind of cancer selected from the group consisting of malignant pleural mesothelioma, lung cancer, gastric cancer and breast cancer.
[19] The agent of any of the above-mentioned [10] - [18], which is a reagent for study.
[20] A method for the prophylaxis and/or treatment of cancer, comprising administering an effective amount of an inositol phospholipid compound to a subject in need thereof.
[21] The method of the above-mentioned [20], wherein the fatty acid constituting the inositol phospholipid compound is an unsaturated fatty acid.
[22] The method of the above-mentioned [21], wherein the unsaturated fatty acid is oleic acid.
[23] The method of the above-mentioned [20], wherein the fatty acid constituting the inositol phospholipid compound is an unsaturated fatty acid having a double bond converted to a cyclopropane ring.
[24] The method of the above-mentioned [23], wherein the unsaturated fatty acid is 8-[2-(2-pentyl-cyclopropylmethyl)-cyclopropyl]-octanoic acid (DCP-LA).
[25] The method of the above-mentioned [20], wherein the fatty acid constituting the inositol phospholipid compound is a saturated fatty acid.
[26] The method of the above-mentioned [25], wherein the saturated fatty acid is palmitic acid.
[27] The method of the above-mentioned [20], wherein the inositol phospholipid compound is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(1'-myo-inositol) (DO-PI), or 1,2-dipalmitoyl-sn-glycerol-3-phospho-(1'-myo-inositol) (DP-PI).
[28] The method of any of the above-mentioned [20] - [27], wherein the treatment target cancer is at least one kind selected from the group consisting of malignant pleural mesothelioma, lung cancer, gastric cancer and breast cancer.
[29] A method of inducing cell death of a cancer cell, comprising treating the cancer cell with an inositol phospholipid compound.
[30] The method of the above-mentioned [29], wherein the fatty acid constituting the inositol phospholipid compound is an unsaturated fatty acid.
[31] The method of the above-mentioned [30], wherein the unsaturated fatty acid is oleic acid.
[32] The method of the above-mentioned [29], wherein the fatty acid constituting the inositol phospholipid compound is an unsaturated fatty acid having a double bond converted to a cyclopropane ring.
[33] The method of the above-mentioned [32], wherein the unsaturated fatty acid is 8-[2-(2-pentyl-cyclopropylmethyl)-cyclopropyl]-octanoic acid (DCP-LA).
[34] The method of the above-mentioned [29], wherein the fatty acid constituting the inositol phospholipid compound is a saturated fatty acid.
[35] The method of the above-mentioned [34], wherein the saturated fatty acid is palmitic acid.
[36] The method of the above-mentioned [29], wherein the inositol phospholipid compound is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(1'-myo-inositol) (DO-PI), or 1,2-dipalmitoyl-sn-glycerol-3-phospho-(1'-myo-inositol) (DP-PI).
[37] The method of any of the above-mentioned [29] - [36], wherein the cancer cell is a cell of at least one kind of cancer selected from the group consisting of malignant pleural mesothelioma, lung cancer, gastric cancer and breast cancer.

### Effect of the Invention

An inositol phospholipid compound, particularly an inositol phospholipid compound having, as a constituting fatty acid, an unsaturated fatty acid, particularly a fatty acid having a double bond substituted by a cyclopropane ring, has a superior cancer cell death inducing action. Phospholipid compounds are inherently present in the living body. Therefore, the present invention can more safely provide a superior anti-cancer agent.

### Brief Description of the Drawings

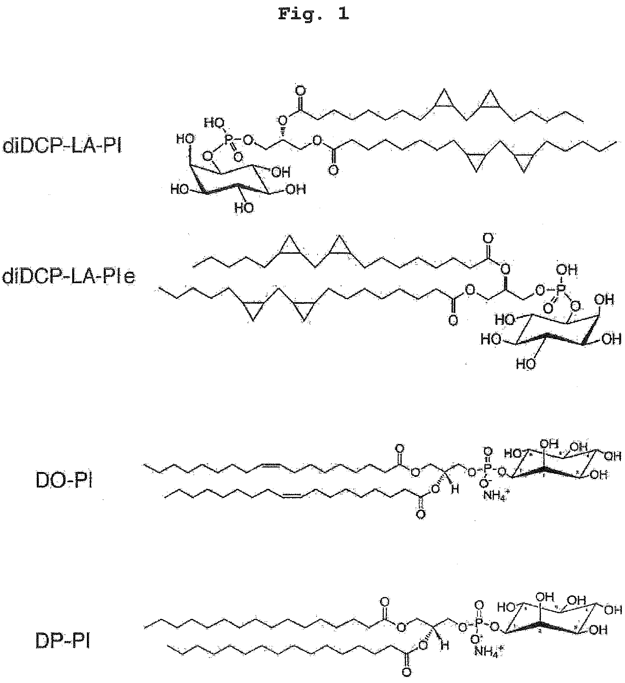

Fig. 1 shows the structure of an inositol phospholipid compound contained as an active ingredient in the present invention. diDCP-LA-PI: 1,2-O-bis-[8-{2-(2-pentyl-cyclopropylmethyl)-cyclopropyl}-octanoyl]-sn-glycerol-3-phosphatidyl-D-inositol, diDCP-LA-PIe: 1,2-O-bis-[8-{2-(2-pentyl-cyclopropylmethyl)-cyclopropyl}-octanoyl]-sn-glycerol-3-phosphatidyl-L-1-inositol, DO-PI: 1,2-dioleoyl-sn-glycerol-3-phospho-(1'-myo-inositol), DP-PI: 1,2-dipalmitoyl-sn-glycerol-3-phospho-(1'-myo-inositol)

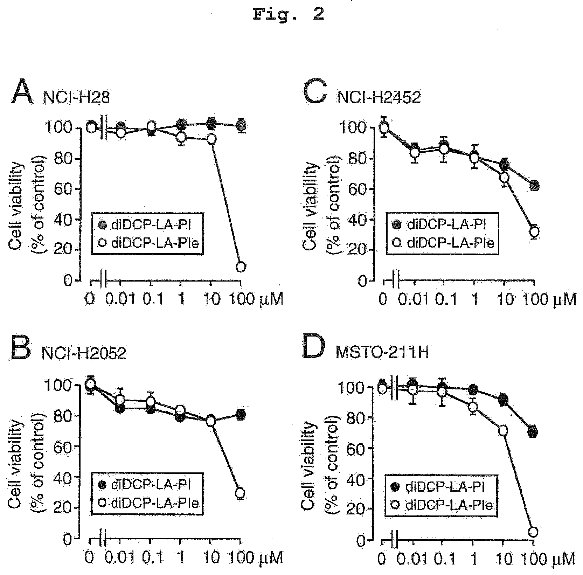

Fig. 2 is a graph showing that diDCP-LA-PI and diDCP-LA-PIe induce cell death of human malignant pleural mesothelioma in a concentration-dependent manner. The cells were treated with a given concentration of diDCP-LA-PI or diDCP-LA-PIe for 48 hr, and subjected to MTT assay. In the graph, each point shows mean (±SD) percentage relative to the MTT intensity of a cell free of a drug treatment (n=4 in each experiment).

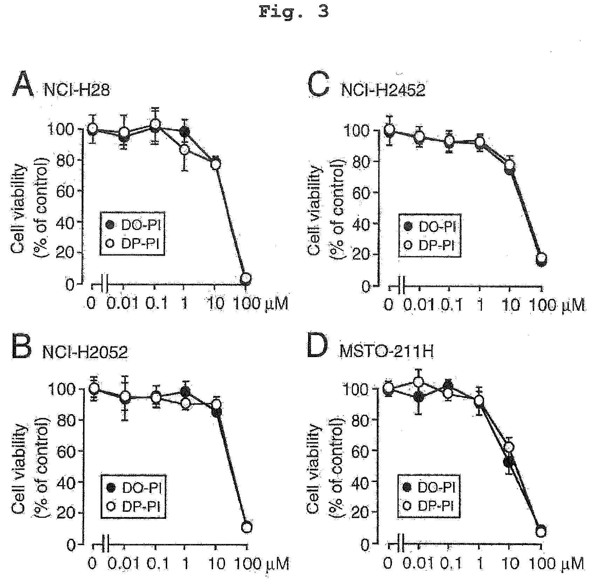

Fig. 3 is a graph showing that DO-PI and DP-PI induce the same level of cell death of human malignant pleural mesothelioma in a concentration-dependent manner. The cells were treated with a given concentration of DO-PI or DP-PI for 48 hr, and subjected to MTT assay. In the graph, each point shows mean (±SD) percentage relative to the MTT intensity of a cell free of a drug treatment (n=4 in each experiment).

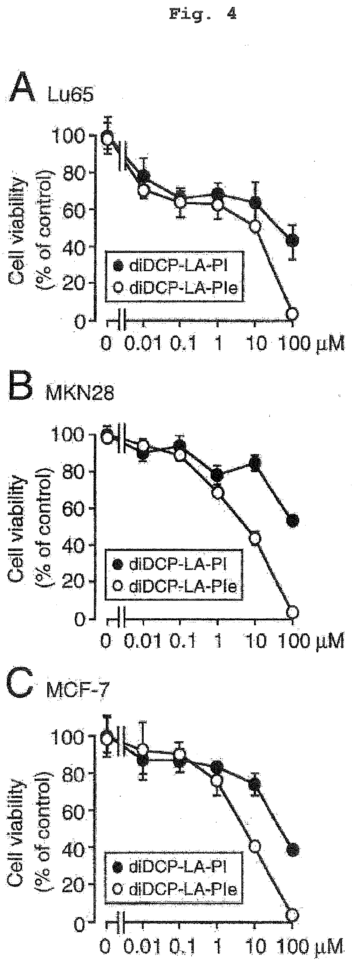

Fig. 4 is a graph showing that diDCP-LA-PI and diDCP-LA-PIe induce cell death of (A) human lung cancer cell (Lu65), (B) gastric cancer cell (MKN28), (C) breast cancer cell (MCF-7) in a concentration-dependent manner. The cells were treated with a given concentration of diDCP-LA-PI or diDCP-LA-PIe for 48 hr, and subjected to MTT assay. In the graph, each point shows mean (±SD) percentage relative to the MTT intensity of a cell free of a drug treatment (n=4 in each experiment).

### Description of Embodiments

The present invention is explained in detail below.

The present invention provides an anti-cancer agent containing an inositol phospholipid compound as an active ingredient (hereinafter to be also referred to as the medicament of the present invention). The inositol phospholipid compound is also referred to as the phospholipid compound of the present invention.

Phospholipid is largely divided into two: glycerophospholipid having glycerol as the skeleton, and sphingophospholipid having sphingosine as the skeleton. It has a structure wherein glycerol or sphingosine is the central skeleton, fatty acid and phosphoric acid are bonded thereto, and alcohol is ester-bonded to phosphoric acid. While the phospholipid compound of the present invention may be any of glycerophospholipid and sphingophospholipid, it is preferably glycerophospholipid. A molecule wherein fatty acid is ester bonded to the C1 and C2-positions of glycerol, and phosphoric acid is ester bonded to the C3-position is called phosphatidic acid.

In the phospholipid compound of the present invention, alcohol that forms an ester bond with phosphoric acid is inositol.

The fatty acid constituting the phospholipid compound of the present invention may be any of saturated fatty acid and unsaturated fatty acid. Specific examples of the saturated fatty acid include lauric acid, myristic acid, pentadecylic acid, palmitic acid, margaric acid and stearic acid. Of these, lauric acid, myristic acid, palmitic acid and stearic acid are preferable, and palmitic acid is more preferable. The unsaturated fatty acid is not particularly limited as long as it is a fatty acid having at least one double bond in a molecule, and may be any of a monovalent unsaturated fatty acid, a polyvalent unsaturated fatty acid, a cis-type unsaturated fatty acid, or a trans-type unsaturated fatty acid.

Examples of the monovalent unsaturated fatty acid include oleic acid, pulmitoleic acid, petroselinic acid, erucic acid, brassidic acid, obtusilic acid, kapurenic acid, undecylenic acid, linderic acid, tsuzuic acid, physeteric acid, myristoleic acid, pulmitoleic acid, elaidic acid, asclepinic acid, vaccenic acid, gadoleic acid, gondoic acid, cetoleic acid, cis-6-hexadecenoic acid and the like.

Examples of the polyvalent unsaturated fatty acid include linoleic acid, linolenic acid, γ-linolenic acid, ricinoleic acid, α-eleostearic acid, β-eleostearic acid, punicic acid, trans-10-octadecadienoic acid, trans-12-octadecadienoic acid and the like.

Preferred are linoleic acid (cis,cis-9,12-octadienoic acid) and oleic acid (cis-9-octadecenoic acid), more preferably linoleic acid.

In the unsaturated fatty acid, its double bond may be converted to a cyclopropane ring, and such embodiment is also recited as a preferable example of the present invention. In the case of a polyvalent unsaturated fatty acid, all double bonds may be converted to cyclopropane rings, and a part of the double bond may be converted to a cyclopropane ring.

Preferred as an unsaturated fatty acid having a double bond converted to a cyclopropane ring in the present invention is 8-[2-(2-pentyl-cyclopropylmethyl)-cyclopropyl]-octanoic acid (DCP-LA) wherein the double bond of linoleic acid is converted to a cyclopropane ring.

It should be noted that the phospholipid compound of the present invention may contain one or more stereoisomers (e.g., optical isomer, geometric isomer) due to an asymmetric carbon atom or a double bond, and all of such isomers and mixtures thereof are encompassed within the scope of the present invention.

The phospholipid compound of the present invention can be synthesized according to a general synthesis method of phospholipid (e.g., the method described in "The Chemical Society of Japan ed., 5th ed., Jikken Kagaku Kouza 16, synthesis of organic compound (kyo); carboxylic acid·amino acid·peptide, chapter 3, phosphate"). More particularly, the compound can be synthesized according to the method described in Examples.

The unsaturated fatty acid having a double bond converted to a cyclopropane ring can be produced, for example, by the method described in WO 02/50013 or a method analogous thereto. Alternatively, it may be extracted from a naturally occurring substance. A production method of DCP-LA is described in WO 02/50013.

The inositol phospholipid compound contained as an active ingredient in the present invention is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(1'-myo-inositol) (DO-PI), or 1,2-dipalmitoyl-sn-glycerol-3-phospho-(1'-myo-inositol) (DP-PI), more preferably diDCP-LA-PIe. The diDCP-LA-PIe is a non-natural type optical isomer of diDCP-LA-PI.

The structure of each phospholipid compound is shown in Fig. 1.

The phospholipid compound of the present invention may also be used in the form of a salt thereof. Such salt is not particularly limited, and a salt acceptable as a medicament or food is preferable. Examples thereof include salts with inorganic base (e.g., alkali metal such as sodium, potassium and the like; alkaline earth metal such as calcium, magnesium and the like; aluminum, ammonium), organic base (e.g., trimethylamine, triethylamine, pyridine, picoline, ethanolamine, diethanolamine, triethanolamine, dicyclohexylamine, N,N-dibenzylethylenediamine), inorganic acid (e.g., hydrochloric acid, hydrobromic acid, nitric acid, sulfuric acid, phosphoric acid), organic acid (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid), basic amino acid (e.g., arginine, lysine, ornithine) or acidic amino acid (e.g., aspartic acid, glutamic acid) and the like.

The phospholipid compound of the present invention has an action to induce cell death of various cancer cells and suppress cell proliferation, as shown in the data of the Examples. Such pharmacological action shows that the phospholipid compound of the present invention is useful as a cancer cell death-inducing agent (hereinafter to be simply referred to as the agent of the present invention), and further, as an anti-cancer agent or a prophylactic or therapeutic drug for cancer (hereinafter to be simply referred to as the medicament of the present invention). In addition, it suggests that the phospholipid compound is also useful as a reagent for study which can be a tool useful for the development of an anti-cancer agent or a prophylactic or therapeutic drug for cancer.

Moreover, by the pharmacological action of the phospholipid compound of the present invention, the present invention can provide a method of inducing the cell death of cancer cells, and a prophylactic and/or treatment method of cancer (hereinafter to be simply referred to as the method of the present invention).

When used in the present specification, the test subject (target) may be a mammal. Examples of the mammal include primates (e.g., human, monkey, chimpanzee), rodents (e.g., mouse, rat, guinea pig), pets (e.g., dog, cat, rabbit), working animals or domestic animals (e.g., bovine, horse, swine, sheep, goat), and human is preferable.

The cancer (cancer cell) to which the agent, medicament or method of the present invention is applied is not particularly limited, and specific examples thereof include glioblastoma, medulloblastoma, cancer of the tongue, pharynx cancer, laryngeal cancer, esophagus cancer, gastric cancer, colorectal cancer, liver cancer, gall bladder cancer, biliary tract cancer, pancreatic cancer, kidney cancer, adrenal cancer, urinary bladder cancer, prostate cancer, penile cancer, uterine cancer, ovarian cancer, vulva cancer, virginal cancer, breast cancer, thyroid cancer, lung cancer, malignant pleural mesothelioma, skin cancer, malignant melanoma, malignant bone tumor, soft tissue sarcoma, malignant lymphoma, leukemia, multiple myeloma and the like. Preferably, they are applied to malignant pleural mesothelioma, lung cancer, gastric cancer and breast cancer.

Similarly, when used in the present specification, the target cancer cells to be treated by the phospholipid compound of the present invention are various cancer cells to which the above-mentioned medicament of the present invention is applied, and preferred are the cells of malignant pleural mesothelioma, lung cancer, gastric cancer and breast cancer. The "treatment" here means bringing the above-mentioned cells into contact with the phospholipid compound of the present invention for a necessary and sufficient time. While the time varies depending on the desired effect and the kind of the cells to be used, it is generally 0.5 - for 76 hr, preferably about 0.5 - for 48 hr. Alternatively, the treatment may be performed for a shorter time, for example, about 0.5 - 24 hr, preferably about 0.5 - 12 hr. Conveniently, it is performed by culturing in a culture medium containing the phospholipid compound of the present invention.

While the dose of the agent or medicament of the present invention varies depending on the kind of cancer of the administration subject, severity thereof, animal species to be the administration subject, drug acceptability, body weight, age and the like of the administration subject, the kind of the inositol phospholipid compound to be administered, the daily dose of the phospholipid compound of the present invention to be the active ingredient is 5 - 50 mg, preferably 5 - 25 mg, for an adult by intravenous administration, the daily dose of the compound is 50 - 500 mg, preferably 50 - 250 mg, for an adult by intramuscular administration, and the daily dose of the compound is 0.5 - 5 g, preferably 0.5 - 2.5 g, for an adult by oral administration, which is generally administered for the prophylaxis and/or treatment of cancer.

The medicament of the present invention can contain, besides the phospholipid compound of the present invention which is the active ingredient, any additive, for example, a pharmaceutically acceptable carrier. Examples of the pharmaceutically acceptable carrier include, but are not limited to, excipients such as sucrose, starch, mannit, sorbit, lactose, glucose, cellulose, talc, calcium phosphate, calcium carbonate and the like, binders such as cellulose, methylcellulose, hydroxypropylcellulose, polypropylpyrrolidone, gelatin, gum arabic, polyethylene glycol, sucrose, starch and the like, disintegrants such as starch, carboxymethylcellulose, hydroxypropylstarch, sodium-glycol-starch, sodium hydrogen carbonate, calcium phosphate, calcium citrate and the like, lubricants such as magnesium stearate, aerosil, talc, sodium lauryl sulfate and the like, aromatic substances such as citric acid, menthol, glycyllysin ammonium salt, glycine, orange powder and the like, preservatives such as sodium benzoate, sodium bisulfite, methylparaben, propylparaben and the like, stabilizers such as citric acid, sodium citrate, acetic acid and the like, suspensions such as methylcellulose, polyvinylpyrrolidone, aluminum stearate and the like, dispersing agents such as surfactant and the like, diluents such as water, saline, orange juice and the like, base waxes such as cacao butter, polyethylene glycol, kerosene and the like, and the like.

In one embodiment, the medicament of the present invention can be formulated as a preparation preferable for oral administration. Examples of the preparation preferable for oral administration include a liquid wherein an effective amount of a substance is dissolved in a diluent such as water and saline, a capsule, granule, powder or tablet containing an effective amount of a substance as a solid or granules, a suspension wherein an effective amount of a substance is suspended in a suitable dispersion medium, an emulsion wherein a solution of an effective amount of a substance is dispersed and emulsified in a suitable dispersion medium, and the like.

In another embodiment, the medicament of the present invention can be formulated as a preparation preferable for parenteral administration. Examples of the preparation preferable for parenteral administration (e.g., intravenous injection, subcutaneous injection, muscular injection, topical injection and the like) include aqueous and nonaqueous isotonic aseptic injection liquids, which may contain antioxidant, buffer, bacteriostatic, isotonicity agent and the like. In addition, examples thereof include aqueous and non-aqueous aseptic suspensions, which may contain suspension, solubilizer, thickener, stabilizer, preservative and the like. Unit dose or plural doses of the preparation can be filled in a container such as ampoule and vial. Moreover, the active ingredient and a pharmaceutically acceptable carrier can be freeze-dried and preserved in a form that can be dissolved or suspended in a suitable aseptic vehicle immediately before use.

The medicament of the present invention may be packed or filled individually by a unit ingestion amount or a divided amount thereof, or packed or filled comprehensively by many unit ingestion amounts or divided amounts thereof.

When the medicament of the present invention is provided as a single preparation, the unit ingestion amount of the medicament or a divided amount thereof is the unit ingestion amount of the whole phospholipid compound of the present invention or a divided amount thereof.

Examples of the medicament wherein a unit ingestion amount or a divided amount thereof is packed or filled individually include general packages (e.g., PTP (press through packing) sheet, paper container, film (e.g., plastic film) container, glass container, plastic container) packed or filled with the unit ingestion amount or a divided amount thereof. The medicaments that are individually packed or filled may be further combined and packed or filled in a single container (e.g., paper container, film (e.g., plastic film) container, glass container, plastic container). Examples of the medicament wherein many unit ingestion amounts or a divided amount thereof are/is comprehensively packed or filled include those wherein many tablets or capsules are packed or filled in a single container (e.g., paper container, film (e.g., plastic film) container, glass container, plastic container) without distinction. The medicament of the present invention may contain a unit ingestion amount or a divided amount thereof in a number sufficient for long-term ingestion. For example, a food can contain same in a number sufficient for ingestion for not less than 3 days, preferably not less than 7 days, 10 days, 14 days or 21 days, or 1 month, 2 months, or not less than 3 months.

The medicament of the present invention may contain, besides the phospholipid compound of the present invention which is an essential active ingredient, one or more kinds of other anti-cancer agents. Examples of other anti-cancer agent include metabolic antagonist (e.g., methotrexate, 5-fluorouracil etc.), alkylating agent (e.g., cyclophosphamide, ifosfamide etc.), platinum anti-cancer agent (e.g., cisplatin, carboplatin etc.), topoisomerase inhibitor (e.g., etoposide etc.), antitumor antibiotics (e.g., mitomycin, adriamycin etc.), plant-derived antitumor agent (e.g., vincristine, vindesine, taxol etc.), tyrosine kinase inhibitor (e.g., gefinitib, imanitib etc.), humanized antibody (e.g., herceptin etc.) and the like.

The contents disclosed in any publication cited in the present specification, including patents and patent applications, are hereby incorporated in their entireties by reference, to the extent that they have been disclosed herein.

While the present invention is explained in more detail in the following by referring to Examples, the present invention is not at all limited by the following Examples and the like.

### Examples

### (Analysis method)

¹H-NMR spectrum was recorded by JEOL JNM-ECX400 spectrometer (400 MHz). As for ¹H-NMR, the chemical shift is shown by values downfielded from TMS (δ=0.00) or CHCl₃ (δ=7.26).

ESI-MS spectrum was measured by Bruker micro TOF-Q mass spectrometer.

Column chromatography was performed on silica gel 60 (40-50 µm and 40-100 µm) (purchased from KANTO CHEMICAL CO., INC.).

All reactions were monitored using UV light, iodine and m-bromocresol green or 5% (w/v) ethanol phosphomolybdate solution and heat as color developing agents on a 0.25 mm silica gel plate 60F254 (Merck, Darmstadt, Germany).

DO-PI and DP-PI were purchased from Avanti Polar Lipids, Inc. (Alabaster, AL, USA).

### Example 1: Synthesis of diDCP-LA-D-PI

To a solution of 1,2-O-bis-[8-{2-(2-pentyl-cyclopropylmethyl)-cyclopropyl}-octanoyl]-sn-glycerol (0.080 g, 0.12 mmol) and triethylamine (0.033 ml, 0.24 mmol) in CH₂Cl₂ (2 ml) was added methyl N,N-diisopropylchlorophosphoramidite (0.028 ml, 0.14 mmol) under ice-cooling. After stirring at room temperature for 10 min, to the mixture were added (-)-2,3,4,5,6-penta-O-benzyl-D-1-inositol (0.11 g, 0.18 mmol) and 1H-tetrazole (0.033 g, 0.48 mmol), 70% (v/v) aqueous solution of tert-butyl peroxide (0.16 ml, 1.2 mmol) was further added, and the mixture was stirred at the same temperature for 20 min. 10% (w/v) aqueous Na₂S₂O₃ solution was added, and the obtained aqueous layer was extracted with CH₂Cl₂. The combined organic layer was dried over anhydrous MgSO₄, and concentrated under reduced pressure. The crude product was purified by silica gel column chromatography (hexane:ethyl acetate=1:1) to give O-(1,2-O-bis-[8-{2-(2-pentyl-cyclopropylmethyl)-cyclopropyl}-octanoyl]-sn-3-glyceryl) O-methyl 0-(2',3',4',5',6'-penta-O-benzyl-D-1'-inositol)phosphate (30 mg, 17%) as a white solid. ¹H-NMR (400 MHz, CDCl₃): δ -0.33- -0.21 (m, 4H), 0.52-0.85 (m, 12H), 0.87-0.95 (m, 6H), 0.96-1.70 (m, 44H), 2.24 (t, J = 7.3 Hz, 2H), 2.26 (t, J = 7.3 Hz, 2H), 3.46-3.54 (m, 2H), 3.67 (d, J = 11.4 Hz, 3H), 3.88 (dd, J = 11.9 and 6.0 Hz, 1H), 3.94 (ddd, J = 6.8, 6.4 and 5.0 Hz, 1H), 4.00-4.15 (m, 4H), 4.24 (ddd, J = 7.7, 7.4 and 2.1 Hz, 1H), 4.34 (t, J = 2.1 Hz, 1H), 4.67 (d, J = 11.4 Hz, 1H), 4.73 (d, J = 11.4 Hz, 1H), 4.75-4.85 (m, 4H), 4.85-4.95 (m, 3H), 4.95 (d, J = 11.4 Hz, 1H), 5.00-5.07 (m, 1H).

To a solution of O-(1,2-0-bis-[8-{2-(2-pentylcyclopropylmethyl)-cyclopropyl}-octanoyl]-sn-3-glyceryl) 0-methyl O-(2',3',4',5',6'-penta-O-benzyl-D-1'-inositol)phosphate (30 mg, 0.020 mmol) in 2-butanone (2 ml) was added NaI (0.017 g, 0.11 mmol). The mixture was stirred at 80°C for 2 hr, 2N HCl was added to the reaction mixture, and the aqueous layer was extracted with chloroform. The organic layer was washed with H₂O and brine, and the combined organic layer was dried over anhydrous MgSO₄, and concentrated under reduced pressure to give 0-(1,2-O-bis-[8-{2-(2-pentyl-cyclopropylmethyl)-cyclopropyl}-octanoyl]-sn-3'-glyceryl) O-methyl O-(2',3',4',5',6'-penta-O-benzyl-D-1'-inositol)phosphate. To a solution of O-(1,2-O-bis-[8-{2-(2-pentyl-cyclopropylmethyl)-cyclopropyl}-octanoyl]-sn-3'-glyceryl) O-methyl O-(2',3',4',5',6'-penta-O-benzyl-D-1'-inositol)phosphate in ethanol (3 ml) was added 10% (w/v) palladium on activated carbon (21 mg). The obtained suspension was stirred under a hydrogen atmosphere (1 atm) at room temperature for 2 hr. The catalyst was removed using a celite pad, rinsed with ethyl acetate, and concentrated under reduced pressure. The obtained crude product was purified by silica gel column chromatography (chloroform:methanol=10:1) to give 1,2-O-bis-[8-{2-(2-pentylcyclopropylmethyl)-cyclopropyl}-octanoyl]-sn-glycero-3-phosphatidyl-D-1-inositol (10 mg, 55%) as a white solid. ¹H-NMR (400 MHz, CDCl₃): δ -0.33- -0.20 (m, 4H), 0.55-0.85 (m, 12H), 0.87-0.95 (m, 6H), 0.96-1.70 (m, 44H), 2.20-2.43 (m, 4H), 3.80-4.51 (m, 5H), 5.18-5.32 (m, 1H); ESI-HRMS (negative ion, sodium formate) calculated for C₄₉H₈₆o₁₃P ([M-H]⁻) 913.5811; found 913.5806.

### Example 2: Synthesis of diDCP-LA-L-PIe

To a solution of 1,2-O-bis-[8-{2-(2-pentylcyclopropylmethyl)-cyclopropyl}-octanoyl]-sn-glycerol (0.175 g, 0.26 mmol) and triethylamine (0.072 ml, 0.52 mmol) in CH₂Cl₂ (5 ml) was added methyl N,N-diisopropylchlorophosphoramidite (0.061 ml, 0.31 mmol) under ice-cooling. After stirring at room temperature for 10 min, to the mixture were added (+)-2,3,4,5,6-penta-O-benzyl-L-1-inositol (0.25 g, 0.39 mmol) and 1H-tetrazole (0.073 g, 1.04 mmol), 70% (v/v) aqueous solution of tert-butyl peroxide (0.34 ml, 2.6 mmol) was further added, and the mixture was stirred at the same temperature for 20 min. 10% (w/v) aqueous Na₂S₂O₃ solution was added, and the obtained aqueous layer was extracted with CH₂Cl₂. The combined organic layer was dried over anhydrous MgSO₄, and concentrated under reduced pressure. The crude product was purified by silica gel column chromatography (toluene:ethyl acetate=6:1) to give O-(1,2-O-bis-[8-{2-(2-pentyl-cyclopropylmethyl)-cyclopropyl}-octanoyl]-sn-3-glyceryl) O-methyl O-(2',3',4',5',6'-penta-O-benzyl-L-1'-inositol)phosphate (90 mg, 25%) as a white solid. ¹H-NMR (400 MHz, CDCl₃): δ -0.33- -0.21 (m, 4H), 0.52-0.85 (m, 12H), 0.87-0.95 (m, 6H), 0.96-1.70 (m, 44H), 2.25 (t, J = 7.3 Hz, 2H), 2.26 (t, J = 7.3 Hz, 2H), 3.46-3.54 (m, 2H), 3.61 (d, J = 11.4 Hz, 3H), 3.94 (dd, J = 11.9 and 6.0 Hz, 1H), 4.00-4.32 (m, 7H), 4.68 (d, J = 11.9 Hz, 1H), 4.72 (d, J = 11.9 Hz, 1H), 4.73-4.85 (m, 8H), 5.15-5.25 (m, 1H).

To a solution of O-(1,2-O-bis-[8-{2-(2-pentylcyclopropylmethyl)-cyclopropyl}-octanoyl]-sn-3-glyceryl) O-methyl O-(2',3',4',5',6'-penta-O-benzyl-L-1-inositol)phosphate (90 mg, 0.070 mmol) in 2-butanone (2 ml) was added NaI (50 mg, 0.33 mmol). After stirring at 80°C for 3 hr, 2N HCl was added to the reaction mixture, and the aqueous layer was extracted with chloroform. The organic layer was washed with H₂O and brine, and the combined organic layer was dried over anhydrous MgSO₄ and concentrated under reduced pressure to give O-(1,2-O-bis-[8-{2-(2-pentyl-cyclopropylmethyl)-cyclopropyl}-octanoyl]-sn-3-glyceryl) O-methyl O-(2',3',4',5',6'-penta-O-benzyl-L-1-inositol) phosphate. To a solution of O-(1,2-O-bis-[8-{2-(2-pentyl-cyclopropylmethyl)-cyclopropyl}-octanoyl]-sn-3-glyceryl) O-methyl O-(2',3',4',5',6'-penta-O-benzyl-L-1-inositol)phosphate in ethanol (5 ml) was added 10% (w/v) palladium on activated carbon (70 mg). The obtained suspension was stirred under a hydrogen atmosphere (1 atm) at room temperature for 2 hr. The catalyst was removed using a celite pad, rinsed with ethyl acetate, and concentrated under reduced pressure. The obtained crude product was purified by silica gel column chromatography (chloroform:methanol=10:1) to give 1,2-O-bis-[8-{2-(2-pentyl-cyclopropylmethyl)-cyclopropyl}-octanoyl]-sn-glycero-3-phosphatidyl-L-1-inositol (20 mg, 43%) as a white solid. ¹H-NMR (400 MHz, CDCl₃): δ -0.33- -0.20 (m, 4H), 0.55-0.85 (m, 12H), 0.87-0.95 (m, 6H), 0.96-1.70 (m, 44H), 2.20-2.43 (m, 4H), 3.50-4.51 (m, 5H), 5.10-5.42 (m, 1H); ESI-HRMS (negative ion, sodium formate) calculated for C₄₉H₈₆O₁₃P ([M-H]⁻) 913.5811; found 913.5811.

### Experimental Example 1: Cancer cell proliferation suppressive action (cancer cell death induction action)

### (Material and method)

### 1. Cell culture

NCI-H28, NCI-H2052, NCI-H2452, and MSTO-211H which are cell lines of human malignant pleural mesothelioma were used. These cells were purchased from American Type Culture Collection (Manassas, VA, USA). The cells were cultured in Roswell Park Memorial Institute (RPMI)-1640 medium added with 0.003% (w/v) L-glutamine.

Lu-65 human lung cancer cell line was purchased from Health Science Research Resources Bank (Osaka, Japan), and cultured in RPMI-1640 medium.

MKN28 human gastric cancer cell line was provided by Dr. Tatematsu (Nagoya University, Japan), and cultured in RPMI-1640 medium.

MCF-7 human breast cancer cell line was obtained from RIKEN cell bank (Ibaraki, Japan) and cultured in Dulbecco's modified Eagle's medium (DMEM).

In all cell cultures, the cells were incubated in a medium added with 10% (v/v) heat-inactivated bovine serum, penicillin (final concentration, 100 U/ml) and streptomycin (final concentration, 0.1 mg/ml) in a humid environment under 5% CO₂ and 95% air at 37°C.

### 2. Cell viability assay

The cell viability was evaluated by an established method using 3-(4,5-dimethyl-2-thiazolyl)-2,5-diphenyl-2H-tetrazolium bromide (MTT).

### (Results)

### 1. Influence of the phospholipid compound of the present invention on human malignant pleural mesothelioma cell line - (1)

Examined using diDCP-LA-PI and diDCP-LA-PIe. The results are shown in Fig. 2.

The results show that diDCP-LA-PI and diDCP-LA-PIe induce cell death of human malignant pleural mesothelioma cells in a concentration dependent manner. Furthermore, it was shown that diDCP-LA-PIe is more effective.

### 2. Influence of the phospholipid compound of the present invention on human malignant pleural mesothelioma cell line - (2)

Examined using DO-PI and DP-PI. The results are shown in Fig. 3.

The results show that DO-PI and DP-PI induce the same level of cell death of human malignant pleural mesothelioma cells in a concentration dependent manner.

### 3. Influence of the phospholipid compound of the present invention on human lung cancer, gastric cancer and breast cancer cell lines

Examined using diDCP-LA-PI and diDCP-LA-PIe. The results are shown in Fig. 4.

The results show that diDCP-LA-PI and diDCP-LA-PIe induce cell death of human lung cancer, gastric cancer and breast cancer in a concentration dependent manner. The results also show that diDCP-LA-PIe is more effective.

### Industrial Applicability

the phospholipid compound of the present invention (inositol phospholipid compound, particularly an inositol phospholipid compound having, as a constituting fatty acid, an unsaturated fatty acid, particularly a fatty acid having a double bond substituted by a cyclopropane ring), has a superior cancer cell death inducing action. Since phospholipid compounds are inherently present in the living body, the present invention can more safely provide a superior anti-cancer agent.

This application is based on patent application No. 2013-121142 filed in Japan (filing date: June 7, 2013), the contents of which are encompassed in full herein.

## Claims

1. An anti-cancer agent comprising an inositol phospholipid compound as an active ingredient.

2. The agent according to claim 1, wherein the fatty acid constituting the inositol phospholipid compound is an unsaturated fatty acid.

3. The agent according to claim 2, wherein the unsaturated fatty acid is oleic acid.

4. The agent according to claim 1, wherein the fatty acid constituting the inositol phospholipid compound is an unsaturated fatty acid having a double bond converted to a cyclopropane ring.

5. The agent according to claim 4, wherein the unsaturated fatty acid is 8-[2-(2-pentyl-cyclopropylmethyl)-cyclopropyl]-octanoic acid (DCP-LA).

6. The agent according to claim 1, wherein the fatty acid constituting the inositol phospholipid compound is a saturated fatty acid.

7. The agent according to claim 6, wherein the saturated fatty acid is palmitic acid.

8. The agent according to claim 1, wherein the inositol phospholipid compound is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(l'-myo-inositol) (DO-PI), or 1,2-dipalmitoyl-sn-glycerol-3-phospho-(1'-myo-inositol) (DP-PI)

9. The agent according to any one of claims 1 to 8, wherein the treatment target cancer is at least one kind selected from the group consisting of malignant pleural mesothelioma, lung cancer, gastric cancer and breast cancer.

10. A cancer cell death-inducing agent comprising an inositol phospholipid compound as an active ingredient.

11. The agent according to claim 10, wherein the fatty acid constituting the inositol phospholipid compound is an unsaturated fatty acid.

12. The agent according to claim 11, wherein the unsaturated fatty acid is oleic acid.

13. The agent according to claim 10, wherein the fatty acid constituting the inositol phospholipid compound is an unsaturated fatty acid having a double bond converted to a cyclopropane ring.

14. The agent according to claim 13, wherein the unsaturated fatty acid is 8-[2-(2-pentyl-cyclopropylmethyl)-cyclopropyl]-octanoic acid (DCP-LA).

15. The agent according to claim 10, wherein the fatty acid constituting the inositol phospholipid compound is saturated fatty acid.

16. The agent according to claim 15, wherein the saturated fatty acid is palmitic acid.

17. The agent according to claim 10, wherein the inositol phospholipid compound is 1,2-O-bis-[8-{2-(2-pentyl-cyclopropylmethyl)-cyclopropyl}-octanoyl]-sn-glycerol-3-phosphatidyl-D-inositol (diDCP-LA-PI), 1,2-O-bis-[8-{2-(2-pentyl-cyclopropylmethyl)-cyclopropyl}-octanoyl]-sn-glycerol-3-phosphatidyl-L-1-inositol (diDCP-LA-PIe), 1,2-dioleoyl-sn-glycerol-3-phospho-(1'-myo-inositol) (DO-PI), or 1,2-dipalmitoyl-sn-glycerol-3-phospho-(1'-myo-inositol) (DP-PI).

18. The agent according to any one of claims 10 to 17, wherein the cancer cell is a cell of at least one kind of cancer selected from the group consisting of malignant pleural mesothelioma, lung cancer, gastric cancer and breast cancer.
